# EUROPÄISCHE PATENTSCHRIFT

(11) **EP 0 700 890 B1**
(45) Veröffentlichungstag und Bekanntmachung des Hinweises auf die Patenterteilung: **01.12.1999**
(21) Anmeldenummer: 95113495.6
(22) Anmeldetag: 28.08.1995
(51) Int. Cl.: C07C 45/41

(54) **Verfahren zur Herstellung von Aldehyden durch katalytische Gasphasenhydrierung von Carbonsäuren oder ihre Derivate mit Hilfe eines Zinnkatalysators**
Process for the preparation of aldehydes by the catalytic vapour phase hydrogenation of carboxylic acids or their derivatives using a tin catalyst
Procédé pour la préparation d'aldéhydes par hydrogénation catalytique en phase gazeuse d'acides carboxyliques ou de leurs dérivés utilisant un catalyseur d'étain

(30) Priorität: 08.09.1994 DE 4431987
(43) Veröffentlichungstag der Anmeldung: 13.03.1996
(73) Patentinhaber: Aventis Research & Technologies GmbH & Co. KG, 65926 Frankfurt am Main (DE)
(72) Erfinder: Weiguny, Jens, Dr., D-64331 Weiterstadt (DE); Borchert, Holger, Dr., D-65929 Frankfurt am Main (DE); Gerdau, Thomas, Dr., D-65817 Eppstein (DE)

(56) Entgegenhaltungen:
- EP-A- 0 539 274
- FR-A- 617 432
- US-A- 4 950 799

## Beschreibung

Die vorliegende Erfindung betrifft ein Verfahren zur Herstellung von aromatischen und aliphatischen Aldehyden durch katalytische Gasphasenhydrierung aromatischer bzw. aliphatischer Carbonsäuren oder ihrer Derivate mit Hilfe eines Zinnkatalysators.

Die Herstellung aromatischer oder aliphatischer Aldehyde durch Reduktion der entsprechenden Carbonsäuren mit molekularem Wasserstoff an verschiedenen Katalysatoren ist bekannt. Die älteste Technik geht auf US 3.935.265 zurück, bei der Alkylester aromatischer Carbonsäuren bei 400 bis 600°C an Aluminiumoxid mit Wasserstoff zu aromatischen Aldehyden umgesetzt werden. Beispielsweise erhält man aus Methylbenzoat Benzaldehyd mit 37 %iger Selektivität bei 39 % Umsatz.
Ferner ist bekannt, daß Zirkondioxid alleine oder dotiert mit Oxiden anderer Metalle wie Chrom, Mangan Eisen, Zink, Kobalt, Bismut, Blei, Rhenium oder Elementen der III. Hauptgruppe wie Bor, Aluminium, Gallium, Indium oder Thallium (EP 150961) oder gemeinsam mit Oxiden von Elementen der Lanthanidgruppe (US 4.328.373) in der Lage ist, Carbonsäuren oder deren Derivate zu den entsprechenden Aldehyden zu hydrieren. Nach US 4.328.373 können ebenso wie Zirkonoxid Oxide des Yttriums, Cers, Praseodyms, Thoriums und Urans auf Aluminiumoxid eingesetzt werden.
EP 573087 beschreibt einen Katalysator, der aus den Oxiden des Aluminiums, Mangans, Zinks und des Kupfers besteht und durch Cofällung der entsprechenden Salze hergestellt wird.

Die Verwendung von Zinnoxiden für die Reduktion von Carbonsäuren zu den korrespondierenden Aldehyden mit Wasserstoff wird in US 4.950.799 und in EP 539274 angegeben. Gemäß US 4.950.799 wurde ein V₂O₅/SnO₂-Mischoxidkatalysator, der auf einen Träger wie z.B. den Oxiden des Aluminiums, Titans, Eisens, Mangans, Zirkons, Chroms, Bleis oder des Kobalts aufgebracht sein kann, zur Reduktion von m-Phenoxybenzoesäure verwendet. Die Selektivität bezogen auf den Aldehyd betrug nur 44 % bei einem Umsatz von 54 %. Die EP 539274 beschreibt die Hydrierung von Carbonsäuren mit einem bimetallischen Katalysator des Typs Ru-Sn-B/*y*-Al₂O₃. So kann z.B. Benzoesäure mit einer Selektivität von 76 % bei einem Umsatz von 76 % zu Benzaldehyd umgesetzt werden. In beiden Fällen liegt die Ausbeute an Aldehyd in einem Bereich, der technisch nicht zu vertreten ist, außerdem werden als Katalysator komplizierte Mehrkomponentensysteme eingesetzt, für deren Herstellung im Falle der EP 539 274 auch teure Ausgangsmaterialien benötigt werden.

Es besteht also ein Bedarf nach einem Verfahren, das die genannten Nachteile vermeidet, mit einem leicht zugänglichen und kostengünstigen Katalysatorsystem arbeitet und die Aldehyde in hoher Ausbeute und Reinheit liefert.

Diese Aufgabe wird gelöst durch ein Verfahren zur Herstellung von Aldehyden durch katalytische Gasphasenhydrierung von Carbonsäuren oder Carbonsäurederivaten bei erhöhter Temperatur, dadurch gekennzeichnet, daß ein Zinnkatalysator eingesetzt wird, der auf einem oxidischen Trägermaterial aufgebracht ist.

Das erfindungsgemäß einzusetzenden Katalysatorsystem kann aus leicht zugänglichen Materialien nach einfachen Verfahren hergestellt werden und liefert für die Hydrierung von Säuren sehr gute Raumzeitausbeuten. Die Verwendung eines komplizierten Mehrkomponentensystems sowie der Einsatz teurer Ausgangsstoffe wie Zirkondioxid und Seltenerdenmetalle wird vermieden.

Als oxidische Trägermaterialien eignen sich z.B. Aluminiumoxid, Zirkonoxid, Eisenoxid, Titanoxid oder Yttriumoxid, wobei Aluminiumoxid aus Kostengründen besonders vorteilhaft ist.

Das erfindungsgemäß einsetzbare Katalysatorsystem kann durch Imprägnierung oder Cofällung sowie anschließende Trocknung und Kalzination hergestellt werden.
Im ersten Fall wird eine Lösung einer geeigneten Zinnverbindung auf den gewählten Träger, der als Oxid oder Hydroxid vorliegen kann, durch Sprühen oder durch Tränkung aufgebracht. Das Zinn kann in den Verbindungen sowohl als Sn(II) als auch als Sn(IV) vorliegen. Geeignete Verbindungen sind z.B. Zinnhalogenide, Zinnsulfate, Zinnoxalate, Zinncarboxylate, Zinnalkoxide, Zinnhydroxide, Dizinn- und Organozinnverbindungen. Die imprägnierten Träger werden anschließend bei 100 bis 150, bevorzugt bei 130°C, getrocknet und bei 400 bis 900°C, bevorzugt bei 500 bis 700°C, kalziniert. Die so hergestellten Katalysatoren können nach den üblichen Verfahren zu Pellets oder Extrudaten weiterverarbeitet werden.
Im zweiten Fall können geeignete Metallsalze der Trägermaterialien und geeignete Zinnverbindungen bei pH-Werten von 6 bis 10 cogefällt werden. Die Wahl der Metallsalze richtet sich nach der Verfügbarkeit der Salze für die entsprechenden Katalysatoren. Beispielsweise wird beim Aluminium das Nitrat bevorzugt, während beim Zirkon das Oxidnitrat oder das Oxiddichlorid geeignet sind. Nach der Fällung werden die Hydroxide abfiltriert und mit einem geeigneten Lösungsmittel gewaschen. Die Trocknung erfolgt bei 100 bis 150°C, bevorzugt bei 130°C, gegebenenfalls unter Anlegung von Vakuum, die Kalzinierung bei 400 bis 900°C, bevorzugt bei 500 bis 700°C. Als Zeit für die Kalzination kommen 2 bis 10 Stunden in Frage. Der so hergestellte Zinnkatalysator liegt als Granulat vor und kann nach Zerkleinerung auf die gewünschte Teilchengröße direkt in die Reaktion eingesetzt werden.

Es hat sich in vielen Fällen bewährt das Zinn in einem atomaren Verhältnis zum Träger von 0.001/1 bis 0.5/1, bevorzugt von 0.005/1 bis 0.2/1, einzusetzen.

Vor der Reduktion der Carbonsäuren ist es vorteilhaft, den Katalysator bei höheren Temperaturen mit einem geeignetem Reduktionsmittel zu präformieren.
Die Hydrierreaktion der Carbonsäuren kann in kontinuierlicher oder absatzweiser Fahrweise durchgeführt werden. Es hat sich als günstig erwiesen bei einer Temperatur von 250 bis 600°C, insbesondere bei 300 bis 400°C, und einem Druck von 0.1 bis 10 bar, insbesondere bei atmosphärischem Druck, zu arbeiten.
Die Hydrierung kann mit molekularem Wasserstoff durchgeführt werden, der auch in situ hergestellt werden kann. Es ist auch möglich den Wasserstoff mit einem Inertgas wie Stickstoff oder Argon zu verdünnen. Die carboxylische Komponente kann als Feststoff, als Schmelze oder als Lösung in einem geeignetem Lösungsmittel, wie Benzol, Toluol, Xylol oder Cyclohexan einem Verdampfer und anschließend in der Gasphase dem erfindungsgemäß einzusetzenden Katalysator zugeführt werden. In vielen Fällen hat es sich bewährt, wenn das molare Verhältnis der carboxylischen Komponente zu Wasserstoff bei 1:1 bis 1:500 liegt, vorzugsweise wird das Verfahren mit einem Verhältnis von 1:5 bis 1:50 durchgeführt. Die Zufuhrgeschwindigkeit für die Carbonsäuren und deren Derivate liegt zweckmäßig bei 0.01 bis 2 g/ml_{cat}*h (LHSV: liquid hourly space velocity), die des Wasserstoffs bei 100 bis 10000 h⁻¹ (GHSV: Gasous hourly space velocity).

Gemäß der vorliegenden Erfindung können aliphatische und aromatische Carbonsäuren und deren Derivate zu den entsprechenden Aldehyden hydriert werden. Als Derivate sind z.B. Ester und Anhydride gut geeignet.

Von großem Interesse ist das Verfahren für carboxylische Verbindungen der Formeln (I) und (II) 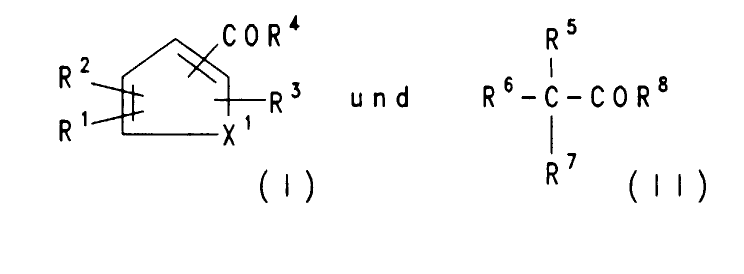 worin
- R¹: Wasserstoff, geradkettig oder verzweigtes C₁-C₈-Alkyl, geradkettiges oder verzweigtes C₁-C₈-Alkoxy, R³-substituiertes Phenyl, Naphthyl, R³-substituiertes Phenoxy, R³-substituiertes Benzyl, R³-substituiertes Benzyloxy, Hydroxy, Amino, NH-(C₁-C₈-alkyl), N-(C₁-C₈-alkyl)₂, Halogen oder COR⁴ ist,
- R²: für Wasserstoff, geradkettiges oder verzweigtes C₁-C₈-Alkyl, geradkettiges oder verzweigtes C₁-C₈-Alkoxy oder R³-substituiertes Phenyl steht,
wobei R¹ und R² gemeinsam einen kondensierten Benzolring bilden können, der mit Hydroxy, Amino, Methyl, Ethyl, Methoxy oder Ethoxy substituiert sein kann,
- R³: Wasserstoff, geradkettiges oder verzweigtes C₁-C₈-Alkyl, geradkettiges oder verzweigtes C₁-C₈-Alkoxy, Hydroxy, Amino oder Halogen sein kann,
- R⁴: Hydroxy, C₁-C₄-Alkoxy, Chlor, Brom oder die Gruppe 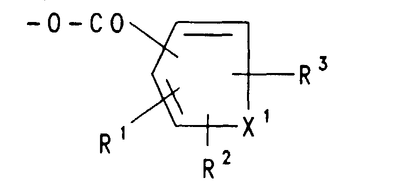 bedeutet,
wobei im letzteren Falle der Anhydridbildung R¹ nicht die Bedeutung COR⁴ annimmt,
- X¹: für -O-, -N-, -S-, -N=CH- oder -CH=CH- steht,
- R⁵: Wasserstoff oder geradkettiges oder verzweigtes C₁-C₈-Alkyl bedeutet,
- R⁶: für Wasserstoff, geradkettiges oder verzweigtes C₁-C₈-Alkyl, R³-substituiertes Phenyl oder Halogen steht,
- R⁷: für Wasserstoff, geradkettiges oder verzweigtes C₁-C₈-Alkyl oder R³-substituiertes Phenyl steht,
wobei das C₁-C₈-Alkyl durch Halogen, Methoxy oder Ethoxy substituiert sein kann und wobei weiterhin R⁶ und R⁷ gemeinsam Dimethylen, Tetramethylen oder Pentamethylen bedeuten können, und
- R⁸: Hydroxy, C₁-C₄-Alkoxy, Chlor, Brom oder die Gruppe -O-CO-C(R⁵,R⁶,R⁷) bedeutet.

Wichtig ist das Verfahren z.B. für Verbindungen der Formel (III), 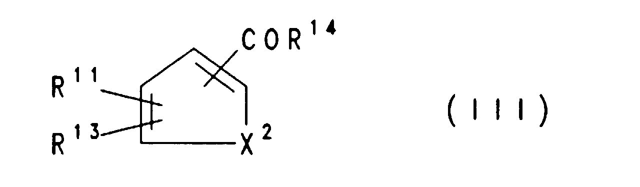 worin
- R¹¹: Wasserstoff, geradkettig oder verzweigtes C₁-C₈-Alkyl, geradkettiges oder verzweigtes C₁-C₈-Alkoxy, R¹³-substituiertes Phenyl,
R¹³-substituiertes Phenoxy, Hydroxy, Amino, NH-(C₁-C₈-alkyl), N-(C₁-C₈-alkyl)₂ oder Halogen bedeutet,
- R¹³: für Wasserstoff, geradkettiges oder verzweigtes C₁-C₈-Alkyl, geradkettiges oder verzweigtes C₁-C₈-Alkoxy, Hydroxy oder Halogen steht,
- R¹⁴: Hydroxy, Methoxy, Ethoxy oder Chlor bedeutet und
- X²: für -CH = CH- oder -N = CH-, bevorzugt für -CH = CH- steht, so daß als aromatische Carbonsäuren oder deren Derivate bevorzugt solche der Formel (IV) 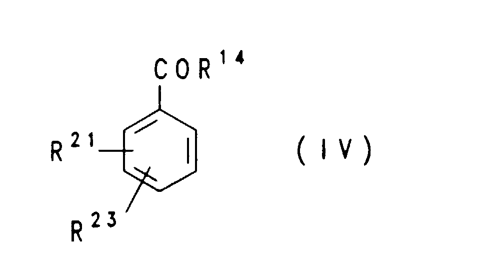 eingesetzt werden, in der
- R²¹: Wasserstoff, geradkettig oder verzweigtes C₁-C₄-Alkyl, geradkettiges oder verzweigtes C₁-C₄-Alkoxy, R²³-substituiertes Phenyl, R²³-substituiertes Phenoxy, Hydroxy, Amino, Methylamino, Ethylamino, Dimethylamino, Diethylamino, Fluor oder Chlor ist,
- R²³: für Wasserstoff, geradkettiges oder verzweigtes C₁-C₈-Alkyl, geradkettiges oder verzweigtes C₁-C₄-Alkoxy, Hydroxy, Fluor oder Chlor steht, und
- R¹⁴: den oben angeführten Bedeutungsumfang hat.

Besondere Bedeutung hat das Verfahren auch für Verbindungen der Formel (V) 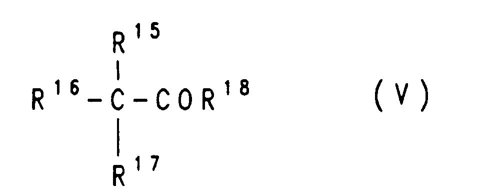 worin
- R¹⁵: für Wasserstoff, Methyl oder Ethyl steht,
- R¹⁶: Wasserstoff, geradkettiges oder verzweigtes C₁-C₈-Alkyl oder Phenyl bedeutet,
- R¹⁷: Wasserstoff, Methyl oder Ethyl bedeutet,
wobei weiterhin R¹⁶ und R¹⁷ gemeinsam Tetramethylen oder Pentamethylen bedeuten können, und
- R¹⁸: für Hydroxy, Methoxy, Ethoxy oder Chlor steht.

Großes technisches Interesse hat das Verfahren, wenn Benzoesäure, Chlorbenzoesäure, Fluorbenzoesäure, Methylbenzoesäure, Methoxybenzoesäure, Phenoxybenzoesäure, tert. Butylbenzoesäure, p-lsopropylbenzoesäure, Pivalinsäure, 6-Methoxy-[2]-naphthoesäure oder deren Methyl- oder Ethylester eingesetzt werden.

Die erfindungsgemäß hergestellten Aldehyde finden als industrielle Zwischenprodukte breite Anwendung bei der Herstellung von Pflanzenschutzmitteln, Pharmaka oder Riechstoffen.

### Beispiele:

### Katalysator A:

Zur Herstellung eines cogefällten Katalysators löst man 361.0 g Al₂O₃*9 H₂O und 12.5 g SnCl₄ in 1.5 l Wasser, kühlt auf 5°C und stellt den pH-Wert der Lösung mit verdünnter Ammoniaklösung auf pH = 9 ein. Der Niederschlag wird abfiltriert und mit Wasser gewaschen. Die Trocknung erfolgt bei 130°C und die Kalzination bei 600°C. Nach Zerkleinerung auf Teilchengrößen von 10 bis 20 mesh wird der Katalysator bei 450°C im Wasserstoffstrom präformiert.

### Katalysator B:

Zur Herstellung eines imprägnierten Katalysators legt man 25 g Al₂O₃ im einem Kolben vor und gibt eine Lösung aus 200 ml Ethanol und 10 g Sn(O^{t}Bu)₄ zu. Nach Durchmischung der Probe wird das Lösungsmittel vorsichtig abdestilliert und der Rückstand bei 600°C kalziniert. Nach Formgebung des Katalysators auf eine Teilchengröße von 10 bis 20 mesh wird der Katalysator bei 450°C im Wasserstoffstrom präformiert.

### Beispiel 1 bis 2:

Die Hydrierungen werden in einem Rohrreaktor durchgeführt. Das Schnittvolumen des Katalysators betrug 20 ml. Die Verdampfung der eingesetzten Benzoesäure erfolgt in einem vorgeschaltetem Verdampfer. GHSV, LHSV und Temperatur werden, wie in Tabelle 1 angegeben, eingestellt. Das Reaktionsgemisch wird mit einem Kühler kondensiert und gaschromatographisch analysiert.

**Tabelle 1**

| Bsp. | Katalysator | GHSV | LHSV | Temperatur | Umsatz | Selektivität |
|---|---|---|---|---|---|---|
| 1 | A | 1250 | 0.13 | 380°C | 69 % | 95 % |
| 2 | B | 1250 | 0.12 | 350°C | 94 % | 92 % |

### Beispiele 3 bis 13:

Die Versuch werden wie in Beispiel 1 bis 2 durchgeführt. Anstelle der Benzoesäure werden andere aromatischen oder aliphatischen Säuren oder deren Derivate eingesetzt. Die Ergebnisse sind in Tabelle 2 angegeben.

**Tabelle 2**

| Bsp. | Kat. | Substrat | GHSV | LHSV | Temp. | Umsatz | Selektivität |
|---|---|---|---|---|---|---|---|
| 3 | A | p-Chlorbenzoesäure | 1250 | 0.12 | 380°C | 99 % | 69 % |
| 4 | B | p-Chlorbenzoesäure | 1250 | 0.08 | 350°C | 97 % | 66 % |
| 5 | B | p-Fluorbenzoesäure | 1250 | 0.10 | 380°C | 95 % | 83 % |
| 6 | B | p-Methoxybenzoesäure | 1250 | 0.08 | 380°C | 96 % | 58 % |
| 7 | B | 6-Methoxy[2]-naphthoesäure | 1250 | 0.14 | 380°C | 90 % | 35 % |
| 8 | B | 3-Phenoxybenzoesäure | 1250 | 0.12 | 380°C | 96 % | 88 % |
| 9 | B | p-Isopropylbenzoesäure | 1250 | 0.06 | 350°C | 99 % | 85 % |
| 10 | B | p-Methylbenzoesäure | 1250 | 0.15 | 380°C | 93 % | 88 % |
| 11 | B | p-tert.-Butylbenzoesäure | 1250 | 0.08 | 350°C | 96 % | 91 % |
| 12 | B | Benzoesäuremethylester | 1250 | 0.07 | 350°C | 91 % | 87 % |
| 13 | B | Pivalinsäure | 1250 | 0.10 | 380°C | 93 % | 82 % |

## Patentansprüche

1. Verfahren zur Herstellung von Aldehyden durch katalytische Gasphasenhydrierung von Carbonsäuren oder Carbonsäurederivaten bei erhöhter Temperatur, dadurch gekennzeichnet, daß ein Zinnkatalysator eingesetzt wird, wobei Mischoxid - oder bimetallische Katalysatoren, die Ruthenium oder Vanadium enthalten, explizit ausgeschlossen sind, der auf einem oxidischen Trägermaterial aufgebracht ist.

2. Verfahren nach Anspruch 1, dadurch gekennzeichnet, daß das Zinn in einem atomaren Verhältnis zum oxidischen Träger von 0,001:1 bis 0,5:1, insbesondere 0,005:1 bis 0,2:1 eingesetzt wird.

3. Verfahren nach Anspruch 1 oder 2, dadurch gekennzeichnet, daß als oxidischer Träger Aluminiumoxid, Zirkonoxid, Eisenoxid, Titanoxid, Yttriumoxid, insbesondere Aluminiumoxid verwendet wird.

4. Verfahren nach mindestens einem der Ansprüche 1 bis 3, dadurch gekennzeichnet, daß die Hydrierung bei einer Temperatur von 250 bis 600°C, insbesondere 300 bis 400°C durchgeführt wird.

5. Verfahren nach mindestens einem der Ansprüche 1 bis 4, dadurch gekennzeichnet, daß die Hydrierung bei einem Druck von 0,1 bis 10 bar, insbesondere 1 bar durchgeführt wird.

6. Verfahren nach mindestens einem der Ansprüche 1 bis 5, dadurch gekennzeichnet, daß das molare Verhältnis von carboxylischer Komponente zu Wasserstoff bei 1:1 bis 1:500, insbesondere 1:5 bis 1:50 liegt.

7. Verfahren nach mindestens einem der Ansprüche 1 bis 6, dadurch gekennzeichnet, daß die Zufuhrgeschwindigkeit für die carboxyllsche Komponente bei 0,01 bis 2 g/ml_{cat}*h, die Zufuhrgeschwindigkeit des Wasserstoffs bei 100 bis 10000 h⁻¹ liegt.

8. Verfahren nach mindestens einem der Ansprüche 1 bis 7, dadurch gekennzeichnet, daß als Carbonsäuren oder Carbonsäurederivate Verbindungen der allgemeinen Formeln (I) und (II) 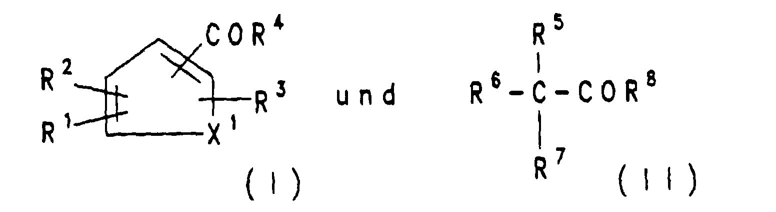 eingesetzt werden, in denen
R¹ Wasserstoff, geradkettig oder verzweigtes C₁-C₈-Alkyl, geradkettiges oder verzweigtes C₁-C₈-Alkoxy, R³-substituiertes Phenyl, Naphthyl, R³-substituiertes Phenoxy, R³-substituiertes Benzyl, R³-substituiertes Benzyloxy, Hydroxy, Amino, NH-(C₁-C₈-alkyl), N-(C₁-C₈-alkyl)₂, Halogen oder COR⁴ ist,
R² für Wasserstoff, geradkettiges oder verzweigtes C₁-C₈-Alkyl, geradkettiges oder verzweigtes C₁-C₈-Alkoxy oder R³-substituiertes Phenyl steht,
wobei R¹ und R² gemeinsam einen kondensierten Benzolring bilden können, der mit Hydroxy, Amino, Methyl, Ethyl, Methoxy oder Ethoxy substituiert sein kann,
R³ Wasserstoff, geradkettiges oder verzweigtes C₁-C₈-Alkyl, geradkettiges oder verzweigtes C₁-C₈-Alkoxy, Hydroxy, Amino oder Halogen sein kann,
R⁴ Hydroxy, C₁-C₄-Alkoxy, Chlor, Brom oder die Gruppe 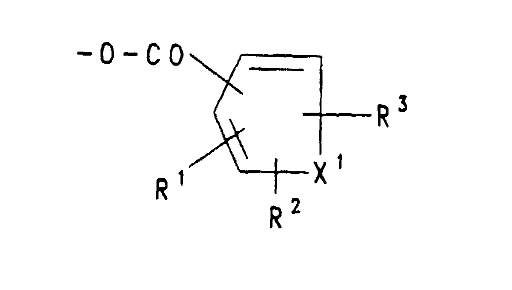 bedeutet,
wobei im letzteren Falle der Anhydridbildung R¹ nicht die Bedeutung COR⁴ annimmt,
X¹ für -O-, -N-, -S-, -N=CH- oder -CH =CH- steht,
R⁵ Wasserstoff oder geradkettiges oder verzweigtes C₁-C₈-Alkyl bedeutet,
R⁶ für Wasserstoff, geradkettiges oder verzweigtes C₁-C₈-Alkyl, R³-substituiertes Phenyl oder Halogen steht,
R⁷ für Wasserstoff, geradkettiges oder verzweigtes C₁-C₈-Alkyl oder R³-substituiertes Phenyl steht,
wobei das C₁-C₈-Alkyl durch Halogen, Methoxy oder Ethoxy substituiert sein kann und wobei weiterhin R⁶ und R⁷ gemeinsam Dimethylen, Tetramethylen oder Pentamethylen bedeuten können, und
R⁸ Hydroxy, C₁-C₄-Alkoxy, Chlor, Brom oder die Gruppe -O-CO-C(R⁵,R⁶,R⁷) bedeutet.

9. Verfahren nach mindestens einem der Ansprüche 1 bis 8, dadurch gekennzeichnet, daß als aromatische Carbonsäuren und deren Derivate Verbindungen der Formel (III) 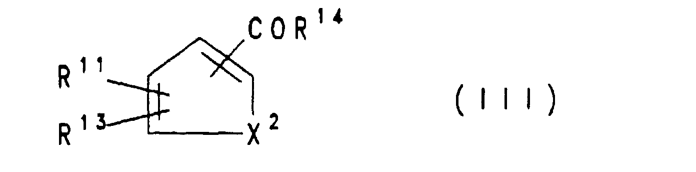 eingesetzt werden, in der
R¹¹ Wasserstoff, geradkettig oder verzweigtes C₁-C₈-Alkyl, geradkettiges oder verzweigtes C₁-C₈-Alkoxy, R¹³-substituiertes Phenyl, R¹³-substituiertes Phenoxy, Hydroxy, Amino, NH-(C₁-C₈-alkyl), N-(C₁-C₈-alkyl)₂ oder Halogen bedeutet,
R¹³ für Wasserstoff, geradkettiges oder verzweigtes C₁-C₈-Alkyl, geradkettiges oder verzweigtes C₁-C₈-Alkoxy, Hydroxy oder Halogen steht,
R¹⁴ Hydroxy, Methoxy, Ethoxy oder Chlor bedeutet und
X² für -CH = CH- oder -N = CH⁻, bevorzugt für -CH = CH- steht, so daß als aromatische Carbonsäuren oder deren Derivate bevorzugt solche der Formel (IV) 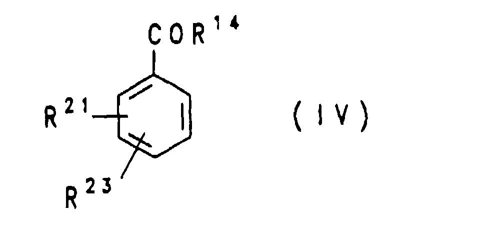 eingesetzt werden, in der
R²¹ Wasserstoff, geradkettig oder verzweigtes C₁-C₄-Alkyl, geradkettiges oder verzweigtes C₁-C₄-Alkoxy, R²³-substituiertes Phenyl, R²³-substituiertes Phenoxy, Hydroxy, Amino, Methylamino, Ethylamino, Dimethylamino, Diethylamino, Fluor oder Chlor ist,
R²³ für Wasserstoff, geradkettiges oder verzweigtes C₁-C₈-Alkyl, geradkettiges oder verzweigtes C₁-C₄-Alkoxy, Hydroxy, Fluor oder Chlor steht, und
R¹⁴ den in Anspruch 9 genannten Bedeutungsumfang hat.

10. Verfahren nach mindestens einem der Ansprüche 1 bis 9, dadurch gekennzeichnet, daß als aliphatische Carbonsäuren und deren Derivaten solche der Formel 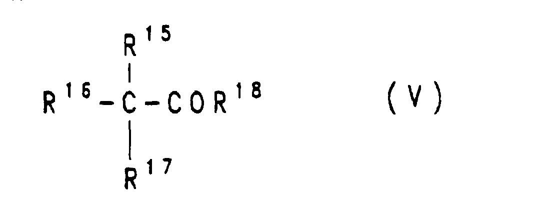 eingesetzt werden, in der
R¹⁵ für Wasserstoff, Methyl oder Ethyl steht,
R¹⁶ Wasserstoff, geradkettiges oder verzweigtes C₁-C₈-Alkyl oder Phenyl bedeutet,
R¹⁷ Wasserstoff, Methyl oder Ethyl bedeutet,
wobei weiterhin R¹⁶ und R¹⁷ gemeinsam Tetramethylen oder Pentamethylen bedeuten können, und
R¹⁸ für Hydroxy, Methoxy, Ethoxy oder Chlor steht.

11. Verfahren nach mindestens einem der Ansprüche 1 bis 10, dadurch gekennzeichnet, daß Benzoesäure, Chlorbenzoesäure, Fluorbenzoesäure, Methylbenzoesäure, Methoxybenzoesäure, Phenoxybenzoesäure, tert.-Butylbenzoesäure, p-lsopropylbenzoesäure, Pivalinsäure, 6-Methoxy-[2]-naphthoesäure oder deren Methyl- oder Ethylester eingesetzt werden.

## Claims

1. A process for the preparation of an aldehyde by catalytic gas phase hydrogenation of a carboxylic acid or carboxylic acid derivative at elevated temperature, which comprises employing a tin catalyst - mixed oxide catalysts or bimetallic catalysts which contain ruthenium or vanadium being explicitly excluded - applied to an oxidic support material.

2. The process as claimed in claim 1, wherein the tin is employed in an atomic ratio to the oxidic support of 0.001:1 to 0.5:1, in particular 0.005:1 to 0.2:1.

3. The process as claimed in claim 1 or 2, wherein aluminum oxide, zirconium oxide, iron oxide, titanium oxide or yttrium oxide, in particular aluminum oxide, is used as the oxidic support.

4. The process as claimed in at least one of claims 1 to 3, wherein the hydrogenation is carried out at a temperature of 250 to 600°C, in particular 300 to 400°C.

5. The process as claimed in at least one of claims 1 to 4, wherein the hydrogenation is carried out under a pressure of 0.1 to 10 bar, in particular 1 bar.

6. The process as claimed in at least one of claims 1 to 5, wherein the molar ratio of carboxylic component to hydrogen is 1:1 to 1:500, in particular 1:5 to 1:50.

7. The process as claimed in at least one of claims 1 to 6, wherein the feed rate for the carboxylic component is 0.01 to 2 g/ml_{cat} × h and the feed rate of the hydrogen is 100 to 10,000 h⁻¹.

8. The process as claimed in at least one of claims 1 to 7, wherein the carboxylic acid or carboxylic acid derivative employed is a compound of the formula (I) or (II) 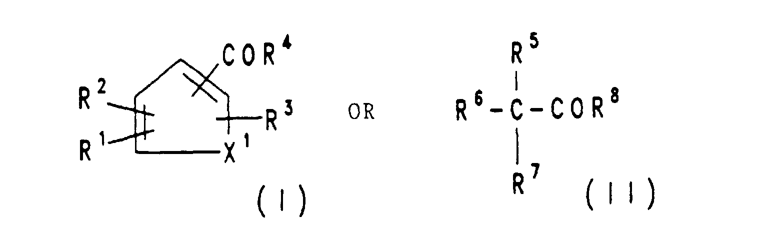 in which
R¹ is hydrogen, straight-chain or branched C₁-C₈-alkyl, straight-chain or branched C₁-C₈-alkoxy, R³-substituted phenyl, naphthyl, R³-substituted phenoxy, R³-substituted benzyl, R³-substituted benzyloxy, hydroxyl, amino, NH-(C₁-C₈-alkyl), N-(C₁-C₈-alkyl)₂, halogen or COR⁴,
R² is hydrogen, straight-chain or branched C₁-C₈-alkyl, straight-chain or branched C₁-C₈-alkoxy or R³-substituted phenyl,
in which R¹ and R² together can form a fused benzene ring, which can be substituted by hydroxyl, amino, methyl, ethyl, methoxy or ethoxy,
R³ can be hydrogen, straight-chain or branched C₁-C₈-alkyl, straight-chain or branched C₁-C₈-alkoxy, hydroxyl, amino or halogen,
R⁴ is hydroxyl, C₁-C₄-alkoxy, chlorine, bromine or the group 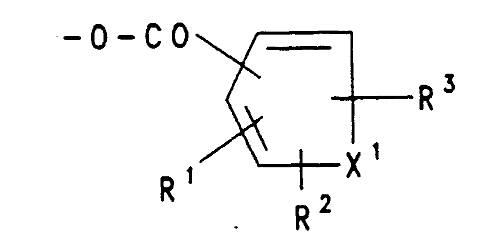 in which, in the latter case of anhydride formation, R¹ does not assume the meaning COR⁴,
X¹ is -O-, -N-, -S-, -N=CH- or -CH=CH-,
R⁵ is hydrogen or straight-chain or branched C₁-C₈-alkyl,
R⁶ is hydrogen, straight-chain or branched C₁-C₈-alkyl, R³-substituted phenyl or halogen,
R⁷ is hydrogen, straight-chain or branched C₁-C₈-alkyl or R³-substituted phenyl,
in which the C₁-C₈-alkyl can be substituted by halogen, methoxy or ethoxy and in which R⁶ and R⁷ furthermore together can be dimethylene, tetramethylene or pentamethylene, and
R⁸ is hydroxyl, C₁-C₄-alkoxy, chlorine, bromine or the group -O-CO-C(R⁵,R⁶,R⁷).

9. The process as claimed in at least one of claims 1 to 8, wherein the aromatic carboxylic acid or derivative thereof employed is a compound of the formula (III) 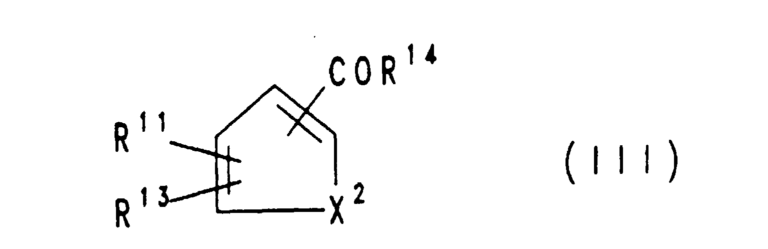 in which
R¹¹ is hydrogen, straight-chain or branched C₁-C₈-alkyl, straight-chain or branched C₁-C₈-alkoxy, R¹³-substituted phenyl, R¹³-substituted phenoxy, hydroxyl, amino, NH-(C₁-C₈-alkyl), N-(C₁-C₈-alkyl)₂ or halogen,
R¹³ is hydrogen, straight-chain or branched C₁-C₈-alkyl, straight-chain or branched C₁-C₈-alkoxy, hydroxyl or halogen,
R¹⁴ is hydroxyl, methoxy, ethoxy or chlorine and
X² is -CH=CH- or -N=CH-, preferably -CH=CH-, so that the aromatic carboxylic acid or derivative thereof employed is preferably one of the formula (IV) 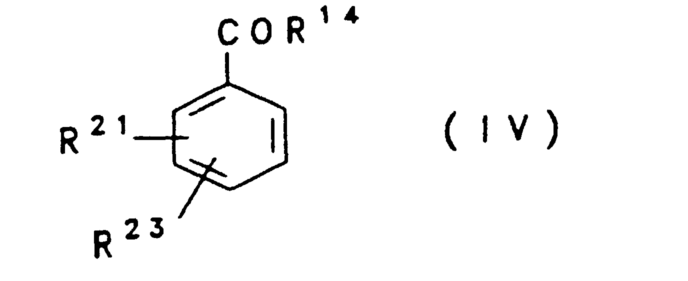 in which
R²¹ is hydrogen, straight-chain or branched C₁-C₄-alkyl, straight-chair or branched C₁-C₄-alkoxy, R²³-substituted phenyl, R²³-substituted phenoxy, hydroxyl, amino, methylamino, ethylamino, dimethylamino, diethylamino, fluorine or chlorine,
R²³ is hydrogen, straight-chain or branched C₁-C₈-alkyl, straight-chain or branched C₁-C₄-alkoxy, hydroxyl, fluorine or chlorine and
R¹⁴ has the scope of meaning given in claim 9.

10. The process as claimed in at least one of claims 1 to 9, wherein the aliphatic carboxylic acid or derivative thereof employed is one of the formula 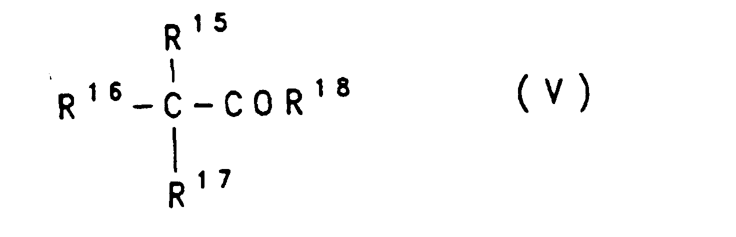 in which
R¹⁵ is hydrogen, methyl or ethyl,
R¹⁶ is hydrogen, straight-chain or branched C₁-C₈-alkyl or phenyl,
R¹⁷ is hydrogen, methyl or ethyl,
in which R¹⁶ and R¹⁷ furthermore together can be tetramethylene or pentamethylene, and
R¹⁸ is hydroxyl, methoxy, ethoxy or chlorine.

11. The process as claimed in at least one of claims 1 to 10, wherein benzoic acid, chlorobenzoic acid, fluorobenzoic acid, methylbenzoic acid, methoxybenzoic acid, phenoxybenzoic acid, tert-butylbenzoic acid, p-isopropylbenzoic acid, pivalic acid, 6-methoxy-[2]-naphthoic acid or the methyl or ethyl ester thereof is employed. to an oxidic support material.

## Revendications

1. Procédé de préparation d'aldéhydes par hydrogénation catalytique en phase gazeuse d'acides carboxyliques ou de dérivés d'acides carboxyliques à température élevée, caractérisé en ce qu'un catalyseur à base d'étain est utilisé, les catalyseurs à base d'oxydes mixtes ou bimétalliques qui contiennent du ruthénium ou du vanadium étant explicitement exclus, lequel catalyseur est déposé sur une matière de support oxydique.

2. Procédé selon la revendication 1, caractérisé en ce que l'étain est utilisé dans une proportion atomique de 0,001:1 à 0,5:1, en particulier de 0,005:1 à 0,2:1 par rapport au support oxydique.

3. Procédé selon la revendication 1 ou 2, caractérisé en ce qu'en tant que support oxydique, on utilise l'oxyde d'aluminium, l'oxyde de zirconium, l'oxyde de fer, l'oxyde de titane, l'oxyde d'yttrium, en particulier l'oxyde d'aluminium.

4. Procédé selon au moins 1-'une des revendications 1 à 3, caractérisé en ce que l'hydrogénation est effectuée à une température de 250 à 600°C, en particulier de 300 à 400°C.

5. Procédé selon au moins l'une des revendications 1 à 4, caractérisé en ce que l'hydrogénation est effectuée sous une pression de 0,1 à 10 bars, en particulier 1 bar.

6. Procédé selon au moins l'une des revendications 1 5 à 5, caractérisé en ce que la proportion molaire du composant carboxylique à l'hydrogène est de 1:1 à 1:500, en particulier de 1:5 à 1:50.

7. Procédé selon au moins l'une des revendications 1 à 6, caractérisé en ce que la vitesse d'alimentation pour le composant carboxylique est de 0,01 à 2 g/ml_{cat}^{∗}h, la vitesse d'alimentation de l'hydrogène est de 100 à 10 000 h⁻¹.

8. Procédé selon au moins l'une des revendications 1 à 7, caractérisé en ce qu'en tant qu'acides carboxyliques ou dérivés d'acides carboxyliques, on utilise des composés de formules générales (I) et (II) 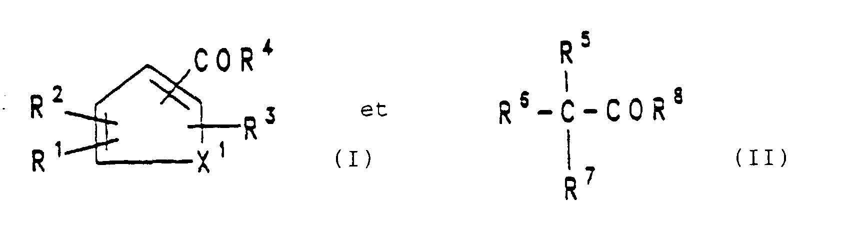 dans lesquelles :
R¹ est hydrogène, alkyle en C₁-C₈ rectiligne ou ramifié, alcoxy en C₁-C₈ rectiligne ou ramifié, phényle substitué par R³, naphtyle, phénoxy substitué par R³, benzyle substitué par R³, benzyloxy substitué par R³, hydroxy, amino, NH-(alkyle en C₁-C₈), N-(alkyle en C₁-C₈)₂, halogène ou COR⁴,
R² représente hydrogène, alkyle en C₁-C₈ rectiligne ou ramifié, alcoxy en C₁-C₈ rectiligne ou ramifié ou phényle substitué par R³,
R¹ et R² pouvant former ensemble un cycle benzène condensé, qui peut être substitué par hydroxy, amino, méthyle, éthyle, méthoxy ou éthoxy,
R³ peut être hydrogène, alkyle en C₁-C₈ rectiligne ou ramifié, alcoxy en C₁-C₈ rectiligne ou ramifié, hydroxy, amino ou halogène,
R⁴ représente hydroxy, alcoxy en C₁-C₄, chlore, brome, ou le groupe 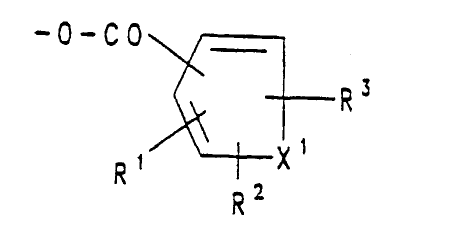 dans le dernier cas de la formation d'anhydride, R¹ ne prenant pas la signification COR⁴,
X¹ représente -O-, -N-, -S-, -N=CH- ou -CH=CH-,
R⁵ représente hydrogène ou alkyle en C₁-C₈ rectiligne ou ramifié,
R⁶ représente hydrogène, alkyle en C₁-C₈ rectiligne ou ramifié, phényle substitué par R³ ou halogène,
R⁷ représente hydrogène, alkyle en C₁-C₈ rectiligne ou ramifié ou phényle substitué par R³,
l'alkyle en C₁-C₈ pouvant être substitué par halogène, méthoxy ou éthoxy et de plus R⁶ et R⁷ pouvant signifier ensemble diméthylène, tétraméthylène ou pentaméthylène, et
R⁸ représente hydroxy, alcoxy en C₁-C₄, chlore, brome ou le groupe -O-CO-C(R⁵,R⁶,R⁷).

9. Procédé selon au moins l'une des revendications 1 à 8, caractérisé en ce qu'en tant qu'acides carboxyliques aromatiques ou leurs dérivés on utilise des composés de formule (III) 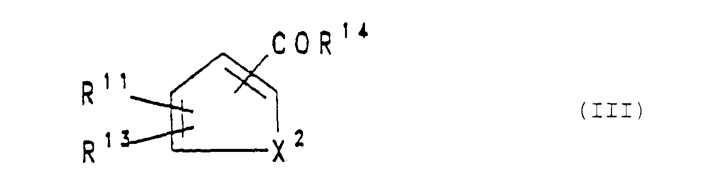 dans laquelle :
R¹¹ représente hydrogène, alkyle en C₁-C₈ rectiligne ou ramifié, alcoxy en C₁-C₈ rectiligne ou ramifié, phényle substitué par R¹³, phénoxy substitué par R¹³, hydroxy, amino, NH-(alkyle en C₁-C₈), N-(alkyle en C₁-C₈)₂ ou halogène,
R¹³ représente hydrogène, alkyle en C₁-C₈ rectiligne ou ramifié, alcoxy en C₁-C₈ rectiligne ou ramifié, hydroxy ou halogène,
R¹⁴ représente hydroxy, méthoxy, éthoxy ou chlore, et
X² représente -CH=CH- ou -N=CH-, de préférence -CH=CH-, de sorte que l'on utilise comme acides carboxyliques aromatiques ou leurs dérivés de préférence ceux qui répondent à la formule (IV) 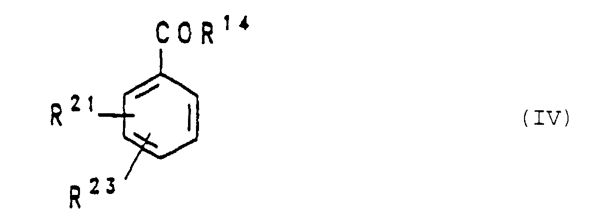 dans laquelle :
R²¹ est hydrogène, alkyle en C₁-C₄ rectiligne ou ramifié, alcoxy en C₁-C₄-rectiligne ou ramifié, phényle substitué par R²³, phénoxy substitué par R²³, hydroxy, amino, méthylamino, éthylamino, diméthylamino, diéthylamino, fluor ou chlore,
R²³ représente hydrogène, alkyle en C₁-C₈ rectiligne ou ramifié, alcoxy en C₁-C₄ rectiligne ou ramifié, hydroxy, fluor ou chlore, et
R¹⁴ a le domaine de significations indiqué dans la revendication 9.

10. Procédé selon au moins l'une des revendications 1 à 9, caractérisé en ce que l'on utilise en tant qu'acides carboxyliques aliphatiques et leurs dérivés , des composés de formule 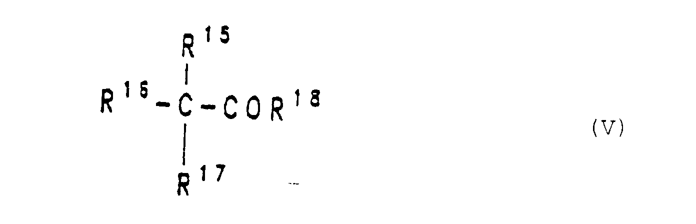 dans laquelle :
R¹⁵ représente hydrogène, méthyle ou éthyle,
R¹⁶ représente hydrogène, alkyle en C₁-C₈ rectiligne ou ramifié ou phényle,
R¹⁷ représente hydrogène , méthyle ou éthyle, de plus R¹⁶ et R¹⁷ pouvant représenter ensemble tétraméthylène ou pentaméthylène , et
R¹⁸ représente hydroxy, méthoxy, éthoxy ou chlore.

11. Procédé selon au moins l'une des revendications 1 à 10, caractérisé en ce qu'on utilise l'acide benzoïque, l'acide chlorobenzoïque, l'acide fluorobenzoïque, l'acide méthylbenzoïque, l'acide méthoxybenzoïque, l'acide phénoxybenzoïque, l'acide tert.-butylbenzoïque, l'acide p-isopropylbenzoïque, l'acide pivalique, l'acide 6-méthoxy-[2]-naphtoïque ou leurs esters méthyliques ou éthyliques.
